# EUROPEAN PATENT APPLICATION

(11) **EP 2 484 348 A2**
(43) Date of publication of application: **08.08.2012**
(21) Application number: 10818996.0
(22) Date of filing: 15.09.2010
(51) Int. Cl.: A61K 31/047, A61K 31/195, A61K 31/7068, A61P 19/02

(54) **PHARMACEUTICAL COMPOSITION FOR TREATING AND PREVENTING DISEASES INVOLVING JOINTS AND CONNECTIVE TISSUE**

(30) Priority: 28.09.2009 KR 20090091763
(71) Applicant: Lee, Il Hoon, Yongin-si, Gyeonggi-do 448-830 (KR); Park, Yoo Sin, Yongin-si, Gyeonggi-do 448-830 (KR)
(72) Inventor: Lee, Il Hoon, Yongin-si, Gyeonggi-do 448-830 (KR); Park, Yoo Sin, Yongin-si, Gyeonggi-do 448-830 (KR)
(74) Representative: Pfenning, Meinig & Partner GbR
(86) International application number: PCT/KR2010/006321
(87) International publication number: WO 2011/037355

(57) **Abstract**

The present invention relates to a pharmaceutical composition for preventing and treating joint-related disease and connective tissue-related disease, the composition comprising two or more selected from the group consisting of polyol, amino acid, ornithine, nucleic acid and vitamin C. The pharmaceutical composition prevents and treats new arthritis, joint damage and connective-related diseases in an effective and economical manner without concerns about side effects, complications and like.

## Description

### Technical Field

The present invention relates to treatment (particularly non-surgical treatment) of joint cartilage diseases, diseases of connective tissue including collagen and the like, joint and connective tissue injuries, and degenerative disc diseases, in mammals.

### Background Art

Collagen is a scleroprotein found in the bone, cartilage, tooth, skin and the like of animals and present as a fibrous solid. It contains amino acids, including proline, hydroxyproline, glycine, glutamine and the like, and is characterized by having a high content of hydroxyproline which is not present in other proteins. Also, hydroxyproline contains a hydroxyl group playing an important role in moisturization and elasticity.

Type-I collagen consists of two α-1(I) polypeptide chains and one α-2 polypeptide chain and is mostly found in skin tissue, bone skeletal structure and the cornea of the eye. Type-II collagen consists of three α-1(II) polypeptide chains and is found mainly in joint cartilage, intervertebral disc and the vitreous body of the eye. Type-III collagen consists of three α-1(III) polypeptide chains and one α-2(IV) polypeptide chain and is found in the placenta and skin.

A joint is a site where two or more bones meet, and it is composed of cartilage, joint capsule, synovial membrane, ligament, tendon, muscle and the like such that it allows the smooth movement between bones. It functions to absorb the impact caused by movement. Arthritis is inflammation of the joint attributable to various causes and typically causes pain in the joint.

Methods for treating arthritis are as follows.

Hyaluronic acid is a component of glycosaminoglycan that is abundantly present in various parts of the body, particularly joints, and it has lubricating and buffering actions in joints. As degenerative changes in joints begin, the concentration of hyaluronic acid in cartilage and synovia decreases and the viscosity of cartilage and synovia decreases, so that the joints are likely to be damaged. When hyaluroniuc acid is injected into the articular cavity, it performs lubricating and buffering functions, thus reducing pain in joints and improving the function of joints. However, because the effect of hyaluronic acid is not long-lasting, it should be repeatedly injected, and when it is repeatedly injected, the amount of hyaluronic acid produced in joints can decrease.

Glucosamine is a component of glycosaminoglycan in cartilage and is found in the matrix of cartilage. It promotes the activity of chondrocyte cells and also reduces proinflammatory substances in joints, thus exhibiting anticancer activity. It was clinically reported that glucosamine reduces pain and delays the degeneration of joints, but such reported effects have not yet been sufficiently verified. Also, continuous administration of glucosamine is required and when the administration thereof is discontinued, the effect thereof is reduced. In addition, chondroitin sulfate, SAM-e, MSM, pentosan polysulfate and the like can assist in reducing the pain of degenerative arthritis, but need to be further studied.

Methods for cartilage repair include surgical methods and non-surgical methods. The surgical methods include: a method in which bone is perforated such that new bone and cartilage grow; a method of transplanting cartilage of other sites; and a method in which autologous chondrocytes are cultured *ex vivo* and then transplanted by a surgical operation. The non-surgical methods include: a method in which undifferentiated stem cells are injected into a cartilage defect site to grow into cartilage; and a method in which cartilage growth factor is injected such that chondrocytes can regenerate by themselves. Cartilage growth factors include TGF-β, IGF-1, BMP-2, etc. When such cartilage growth factors are injected or the secretion thereof is promoted, they promote the growth of cartilage while inhibiting factors interfering with cartilage growth, thereby regenerating cartilage. However, such methods require a considerable amount of cost and cannot achieve sufficient cartilage regeneration. For this reason, artificial joint surgery is currently regarded as the most excellent method, but has shortcomings in that it imposes mental and economical burdens and in that a rehabilitation process after surgery is very difficult and lengthy.

For rheumatoid arthritis, drugs that directly regulate cell response inducers, such as cytokines having potent effects on inflammation, are used. This shows excellent effects after treatment, but has shortcomings in that the symptoms can recur with the passage of time and complications can occur.

The arthritis treatment drugs and methods as described above have problems in terms of lasting effects, complications, economical efficiency, etc., and thus there is an urgent need to develop arthritis treatment agents or methods that overcome such shortcomings.

Meanwhile, connective tissue is tissue that connects different parts and organs of the body, and examples thereof include intervertebral discs, skin, etc.

The vertebrae are composed of 7 cervical vertebrae, 12 thoracic vertebrae, 5 lumbar vertebrae, sacral vertebrae, and coccygeal vertebrae, and have an S-shape when viewed laterally in order to effectively resist atmospheric pressure and body weight. In the vertebrae, an intervertebral disc is present between the vertebrae (excluding cervical vertebrae 1 and 2) to absorb an impact on the vertebrae and to allow various motions of the vertebrae, including flexion, rotation and lateral bending motions. Unlike the vertebrae composed of bone, the discs are composed of the jelly-like semi-solid nucleus pulposus tissue and the annulus fibrous tissue surrounding it. The nucleus pulposus tissue has a moisture content of about 80% for young people, and its moisture content decreases with age, so that the volume and elasticity of the disc decrease, leading to a decrease in the resistance to compression. For this reason, in old persons, the waist is stiff and has reduced flexibility.

Due to aging, the nucleus pulposus tissue is dehydrated to become hard, and the annulus fibrous tissue becomes weaker and is partially cleaved. Thus, when the body lifts heavy things with rotation in a state in which the waist is bent (that is, secondary mechanical stress is applied), or when the body is kept in a poor position, the hardened nucleus pulposus tissue will be extruded through the weakened annulus fibrous tissue or it will rupture the annulus fibrous tissue while a portion of the nucleus pulposus tissue will be released into the spinal cavity. This phenomenon is referred to as disc herniation. If the nucleus pulposus tissue released into the spinal cavity directly compresses the nerve going down the leg, pain will occur. Of course, disc bulging is also frequently observed in which the nucleus pulposus tissue is extruded in all directions to compress nerves.

Methods for treating herniated intervertebral disc disease are as follows. First, physical therapy is relatively easy to perform, but it aims to relieve the symptoms rather than treating intervertebral disc disease itself. Traction therapy is effective in the case in which herniated intervertebral disc disease relatively recently occurred (so-called soft disc herniation), but in other cases, it mainly aims to relieve the symptoms. In addition, deep thermal therapy (sonication, etc.) also has a direct influence on herniated intervertebral disc, but has a poor effect on old herniated intervertebral disc.

In addition, there is drug therapy that uses anti-inflammatory agents, muscle relaxants, steroids, etc. However, this method mainly aims to relieve the symptoms, like physical therapy, and has a problem in that the long-term administration of drugs is difficult due to various side effects caused by the drugs.

Also, neural therapy is performed and is advantageous in that it can exhibit effects within a relatively short time. However, it has shortcomings in that it is highly risky and is highly likely to cause side effects.

Recently, reinforcement therapy has been performed, but it is an indirect approach of treating ligaments and tendons rather than treating the intervertebral disc itself.

Finally, there can be surgical therapy. Examples of the surgical therapy include disc nucleoplasty, chemonucleolysis, discectomy, artificial disc replacement, etc. However, it has various problems, including burdens of surgery, side effects, complications, burdens of cost, and long recovery time. In addition, it aims to remove or lyse the intervertebral disc rather than regenerating the connective tissue of the intervertebral disc. Thus, it does not aim to regenerate the intervertebral disc.

Accordingly, there is a very high demand for a therapeutic method that can regenerate the intervertebral disc using a pharmaceutical agent that is administered orally or by injection in a relatively simple and safe manner.

### Disclosure

### Technical Problem

Accordingly, the present invention has been made keeping in mind the above problems occurring in the prior art, and an object of the present invention is to develop a novel pharmaceutical agent for preventing and treating joint-related diseases and connective tissue-related diseases.

### Technical Solution

In order to accomplish the above object, in accordance with one aspect of the present invention, there is provided a pharmaceutical composition for preventing and treating joint-related diseases and connective tissue-related diseases, the composition comprising two or more selected from the group consisting of polyol, amino acid, ornithine, nucleic acid and vitamin C.

In the composition of the present invention, the concentration of the polyol in the composition may be 2.5-50%, and the polyol may be contained in an amount of 10-90 parts by weight based on 100 parts by weight of the pharmaceutical composition.

The amino acid may be one or more selected from the group consisting of proline (Pro), glycine (Gly), glutamine (Gln), alanine (Ala), aspartic acid (Asp), serine (Ser), glutamic acid (Glu), methionine (Met), lysine (Lys), cysteine (Cys), hydroxyproline (hydroxy-Pro) and arginine (Arg). Also, the concentration of the amino acid in the composition may be 2.5-50%, and the amino acid may be contained in an amount of 10-90 parts by weight based on 100 parts by weight of the pharmaceutical composition.

The amino acid may comprise, based on the total weight of the amino acid, 5-95 parts by weight of proline, 5-95 parts by weight of hydroxyproline, 5-95 parts by weight of glycine, 1-50 parts by weight of glutamine, 1-30 parts by weight of alanine, 1-30 parts by weight of aspartic acid, 1-30 parts by weight of serine, 5-95 parts by weight of arginine, 1-50 parts by weight of methionine, 1-30 parts by weight of cysteine, 1-30 parts by weight of lysine, and 1-30 parts by weight of glutamic acid.

Also, the amino acid may comprise, based on the total weight of the amino acid, 5-85 parts by weight of proline, 5-85 parts by weight of hydroxyproline, 10-90 parts by weight of glycine, 1-30 parts by weight of glutamine, 1-20 parts by weight of alanine, 1-20 parts by weight of aspartic acid, 1-20 parts by weight of serine, 5-80 parts by weight of arginine, 1-30 parts by weight of methionine, 1-20 parts by weight of cysteine, 1-20 parts by weight of lysine, and 1-20 parts by weight of glutamic acid.

Also, the amino acid may comprise, based on the total weight of the amino acid, 5-70 parts by weight of proline, 5-70 parts by weight of hydroxyproline, 10-70 parts by weight of glycine, 1-20 parts by weight of glutamine, 1-10 parts by weight of alanine, 1-10 parts by weight of aspartic acid, 1-10 parts by weight of serine, 5-60 parts by weight of arginine, 1-15 parts by weight of methionine, 1-10 parts by weight of cysteine, 1-10 parts by weight of lysine, and 1-10 parts by weight of glutamic acid.

The concentration of the ornithine in the composition may be 2.5-65%, and the ornithine may be contained in an amount of 10-90 parts by weight based on 100 parts by weight of the pharmaceutical composition.

The nucleic acid may comprise at least one of purine-based nucleic acid-associated substances and pyrimidine-based nucleic acid-associated substances. Also, the concentration of the nucleic acid in the composition may be 0.1-50%, and the nucleic acid may be contained in an amount of 10-90 parts by weight based on 100 parts by weight of the pharmaceutical composition.

Herein, the molecular weight of the nucleic acid may be 100,000-50,000,000 Da.

The purine-based nucleic acid-associated substance may be one or more selected from the group consisting of adenine, adenosine, phosphoric esters of adenosine, metabolic products of phosphoric esters of adenosine, and salts thereof; and guanine, guanosine, phosphoric esters of quanosine, metabolic products of phosphoric esters of guanosine, and salts thereof.

Also, the pyrimidine-based nucleic acid-associated substance may be one or more selected from the group consisting of cytosine, cytidine, phosphoric esters of cytidine, deoxy cytidine, phosphoric esters of deoxy cytidine, and salts thereof; uracil, uridine, phosphoric esters of uridine, metabolic products of phosphoric esters of uridine, and salts thereof; and thymine, thymidine, phosphoric esters of thymidine, orotic acid, 5'-orothidic acid, and salts thereof.

The vitamin C may include a vitamin C derivative. Also, the concentration of the vitamin C in the composition may be 0.01-10%, and the vitamin C may be contained in an amount of 0.1-50 parts by weight based on 100 parts by weight of the pharmaceutical composition.

The vitamin C derivative may be one or more selected from the group consisting of an alkyl ester of L-ascorbic acid, a phosphate of L-ascorbic acid, a sulfate of L-ascorbic acid, and a sodium salt of L-ascorbic acid.

In accordance with another aspect of the present invention, there is provided a pharmaceutical composition for preventing and treating joint-related disease and connective tissue-related disease, the composition comprising: 20-80 parts by weight of polyol whose concentration in the composition is 2.5-50%; 20-80 parts by weigh of ornithine whose concentration in the composition is 2.5-50%; 20-80 parts by weight of amino acid whose concentration in the composition is 2.5-50%, the amino acid being one or more selected from the group consisting of proline, glycine, glutamine, alanine, aspartic acid, serine, glutamic acid, methionine, lysine, cysteine, hydroxyproline and arginine; 20-80 parts by weight of nucleic acid whose concentration in the composition is 0.1-50%; and 0.1-15 parts by weight of vitamin C whose concentration in the composition is 0.01-10%.

The molecular weight of the nucleic acid may be 100,000-50,000,000 Da.

### Advantageous Effects

According to the present invention, a drug having effects against joint damage and connective tissue damage is prepared by mixing a polyol that has the effect of moisturizing connective tissue, an amino acid (e.g., glycine, proline, hydroxyproline, etc.) that is a fundamental component of cartilage and connective tissue, nucleic acid, vitamin C that exhibit antioxidant activity to prevent cartilage degeneration, and the like. When this drug is injected into damaged into damaged and degenerated cartilage, chondrocytes will be regenerated to repair cartilage tissue. This likewise applies to connective tissue.

It is known that, as the extracelluar osmolarity of an intervertebral disk increases, the expression of aggrecan and collagen type II in the intervertebral disk increases. When the pharmaceutical composition according to the present invention is injected into damaged cartilage, the synthesis of aggrecan and collagen type II can be increased to prevent joint- and connective tissue-related diseases.

According to the present invention, arthritis, joint injury and connective tissue diseases can be effectively and economically prevented and treated without concerns about side effects and complications.

### Description of Drawings

FIG. 1 is a set of photographs showing the results of histopathological observation of the femur medial condyle in a normal group, a test group and a control group.
FIG. 2 is a set of photographs showing the results of histopathological observation of the femur lateral condyle in a normal group, a test group and a control group.
FIG. 3 is a set of photographs showing the results of histopathological observation of the tibia medial plateau in a normal group, a test group and a control group.
FIG. 4 is a set of photographs showing the results of histopathological observation of the tibia lateral plateau in a normal group, a test group and a control group.

### Best Mode

Hereinafter, the present invention will be described in further detail.

In accordance with one aspect of the present invention, there is provided a pharmaceutical composition for preventing and treating joint-related disease and connective tissue-related disease, comprising two or more selected from the group consisting of polyol, amino acid, ornithine, nucleic acid and vitamin C.

In the present invention, polyol serves to assist in the recovery of fibrous matter in a tendon, a ligament and a joint and maintain moisturization, thereby maintain the elasticity of joint and connective tissues and relieving pain. Also, polyol functions to stabilize omitine when the omitine is used at high concentration.

Examples of polyol that may be used in the present invention include, but are not limited to, mannitol, erythritol, isomalt, lactitol, maltitol, sorbitol, xylitol, dulcitol, ribitol, inositol, glycerol, molasses, polyethylene glycol, glucose, propylene glycol, invert sugar, 1,3-butylene glycol, isopropyl alcohol, polyvinyl alcohol, polyvinyl pyrrolidine, sorbitane monooleate, etc.

The concentration of the polyol in the composition may be 2.5-50%, and the polyol may be contained in an amount of 10-90 parts by weight based on 100 parts by weight of the pharmaceutical composition.

The hydroxymethyl chain and hydroxyl group of the polyol function to stabilize the stricture of collagen by protein hydration. Thus, when the polyol is introduced into a joint, it can maintain the moisturizing ability and stability of the joint and connective tissues.

Although polyol such as sorbitol or mannitol has been used mainly as drug carriers, it is used as one component of a pharmaceutical composition in the present invention.

This polyol acts to produce an extracellular matrix by stimulating the transcription of TGF-β, ERK and PKC (protein kinase C) through metabolic pathways.

Generally, polyols have a hydroxyl group (-OH) which provides excellent moisturizing ability and elasticity. For example, when polyvinyl alcohol among polyols is administered into an injured joint, it will act as a viscoelastic, biocompatible hydrogel that mimics the original joint cartilage. Also, it promotes the adhesion and growth of chondrocytes by covalent binding between the hydrogel surface and the cell-binding protein fibronectin.

Also, polyol is involved in the formation of protein structures and the interaction between a solvent and a protein to enhance hydrophobicity, thereby stabilizing protein structures. In addition, the hydroxyl group (OH) and hydroxymethyl group (CH₂OH) of polyol contribute to the stabilization of collagen. For example, when polyol such as erythritol, xylitol or sorbitol coexists with collagen, it can provide collagen against guanidine hydrochloride (GdmCl). Thus, the polyol is effective in the prevention and treatment of relevant diseases and diseases related to connective tissue, such as a tendon or a ligament.

Also, pluronic polyol is known to promote wound healing by promoting the early attachment of human gingival fibroblasts even in small amounts and increasing the growth rate of human gingival fibroblasts.

The amino acid that is used in one aspect of the present invention is used to synthesize collagen, and the kind of amino add that may be used in the present invention is not limited. However, amino acids that are involved in growth factors may be selected and used in the present invention. For example, one or more selected from the group consisting of proline (Pro), glycine (Gly), hydroxyproline (hydroxy-Pro), arginine (Arg), aspartic acid (Asp), glutamine (Gln), alanine (Ala), serine (Ser), glutamic acid (Glu), methionine (Met), lysine (Lys) and cysteine (Cys) may be used.

In the present invention, the concentration of the amino acid in the composition may be 2.5-50%, and the amino acid may be contained in an amount of 10-90 parts by weight based on 100 parts by weight of the pharmaceutical composition.

The amino acids that are used in the present invention can act as a substrate for collagen and can influence gene expression.

In addition to the amino acids as described above, other amino acids, including asparagine (Asn), isoleucine (Ile), leucine (Leu), phenylalanine (Phe), trypthophane (Trp), threonine (Thr), histidine (His) and valine (Val), may also be used. Phenylalanine acts on the central nervous system to make feel better, and thus it is used to treat neurological diseases such as melancholia and schizophrenia, as well as obesity. Also, it functions to control pain, and thus can relieve pain caused by magraine, dysmenorrhea or arthritis. In addition, threonine plays an important role in the synthesis of collagen in human connective tissue and the synthesis of elastin in ligaments.

The amino acid that is used in the present invention may be L-amino acid.

Also, the amino acids may be used alone or in a mixture of two or more thereof. When the amino acids are used in a mixture of two or more, the content thereof in the pharmaceutical composition can be suitably determined by a person skilled in the art depending on the amino acid composition and concentration of type II collagen.

For example, the ratio of each amino acid in a mixture of amino acids can be set as follows: proline 5-95%, hydroxyproline 5-95%, glycine 5-95%, glutamine 1-50%, alanine 1-30%, aspartic acid 1-30%, serine 1-30%, arginine 5-95%, methionine 1-50%, cysteine 1-30%, lysine 1-30%, and glutamic acid 1-30%.

Alternatively, the ratio of each amino acid in a mixture of amino acids can be as follows: proline 5-85%, hydroxyproline 5-85%, glycine 10-90%, glutamine 1-30%, alanine 1-20%, aspartic acid 1-20%, serine 1-20%, arginine 5-80%, methionine 1-30%, cysteine 1-20%, lysine 1-20%, and glutamic acid 1-20%.

Alternatively, the ratio of each amino acid in a mixture of amino acids can be as follows: proline 5-70%, hydroxyproline 5-70%, glycine 10-70%, glutamine 1-20%, alanine 1-10%, aspartic acid 1-10%, serine 1-10%, arginine 5-60%, methionine 1-15%, cysteine 1-10%, lysine 1-10%, and glutamic acid 1-10%.

When the amino acids are used in a mixture of two or more, commercially available amino acids are mixed at the above-specified ratio. The composition of the present invention may be prepared or stored at room temperature or below, but the preparation and storage temperature is not specifically limited.

Particularly, proline, hydroxyproline, glycine, glutamine, alanine, aspartic acid, serine, arginine, methionine and cysteine, which are amino acids of collagen, are significant in the pharmaceutical composition for preventing and treating joint- and connective tissue-related diseases.

Among them, proline is converted into the collagen-specific amino acid hydroxyproline by a biochemical process with aid of vitamin C. Hydroxyproline functions to maintain the structure and moisture-absorbing ability of collagen, and thus plays an essential role in maintaining elasticity.

Arginine is known to be effective in enhancing macrophage activity and NK (natural killer) cell activity and increasing interleukin-2 (IL-2) production. Also, arginine is known to promote wound healing, particularly collagen synthesis, and arginine and ornithine show a synergistic effect on wound healing.

Also, arginine (Arg) is known to stimulate IGF-I production and collagen synthesis in osteoblast-like cells, in which IGF-I is an important regulator in osteoblastic metabolism *in vivo* and *in vitro.*

Aspartic acid shows the effect of increasing collagen synthesis to stimulate cells in connective tissue. Particularly, aspartic acid together with hydorxyproline acts on fibroblasts to induce cytostimulation, thus increasing the synthesis of collagen and elastin proteins. Also, aspartic acid synthesizes aspargine by the action of aspartase to promote collagen synthesis.

Glutamine functions to improve immune function and prevent the degradation of muscle fibers. Also, it is a component of glucosamine which constitutes cartilage together with chondroitin and collagen, in which glucosamine is a component of proteoglycan which stimulates chondrocytes to produce new collagen. Such glucosamine exhibits excellent effects against osteoarthritis. Also, glutamine is known to indirectly control the expression of collagen gene in cultured human fibroblasts.

Serine acts as a natural moisturizing factor (NMF) to supply external moisture to cells. Also, methionine is required for DNA-RNA structure, collagen, and cell's protein synthesis, and it is known to stimulate the synthesis of proteoglycan in human joint chondrocytes. Also, the elasticity of body tissue and skin is influenced by cysteine which functions to reduce the abnormal crosslinking of collagen or nervous tissue protein. Also, cysteine is known to activate EPK downstream of MAPK in bovine and human joint chondrocytes.

In one embodiment of the present invention, the pharmaceutical composition for preventing and treating joint-related disease and connective tissue-related disease may comprise ornithine.

Ornithine is a kind of basic amino acid which is contained in protein. Ornitine is an intermediate metabolite of amino acid metabolism and is a component of the urea cycle, which converts ammonia to urea in the liver. Also, ornithine is a raw material for polyamines such as putrescine or spermidine, in which the polyamine influences protein synthesis or cell division by binding to DNA and controlling the replication of DNA.

Also, ornithine is the important metabolite of arginine in the urea cycle and shares many of the biopharmacologic effects of arginine. Thus, ornithine is also known to promote wound healing and collagen synthesis. More specifically, omitine is a precursor of proline which acts in wound healing. Particularly, ornitine and arginine show a synergistic effect on wound healing.

The concentration of ornithine in the composition may be 2.5-65%, and the ornithine may be contained in an amount of 10-90 parts by weight based on 100 parts by weight of the pharmaceutical composition.

However, if ornithine is present at high concentration in the composition, the molecular stability of ornithine is likely to decrease. In this case, polyol such as sorbitol is added, the hydroxyl group of the polyol will increase the molecular stability of ornithine. Thus, if a high concentration of orthine is used, the presence of polyol will be significant.

For wound healing, a significant amount of collagen should be locally synthesized. As an intermediate precursor for collagen synthesis, amino acid is used. Herein, proline and its metabolic precursors, including ornithine, glutamine and glutamic acid, play an important role.

Nucleic acids are linear polymers composed of monomer units called nucleotides linked to each other in a chain and are classified into RNA and DNA. Also, nucleotides are composed of ribose, bases and phosphoric acid, in which the bases include adenine, guanine (purine), cytosine, uracil and thymine (pyrimidine).

The nucleic acid that is used in the present invention may comprise at least one of purine-based nucleic acid-associated substances and pyrimidine-based nucleic acid-associated substances.

The purine-based nucleic acid-associated substances include adenine, adenosine, phosphoric esters of adenosine, metabolic products of phosphoric esters of adenosine, and salts thereof; and guanine, guanosine, phosphoric esters of quanosine, metabolic products of phosphoric esters of guanosine, and salts thereof.

Also, the pyrimidine-based nucleic acid-associated substances may include cytosine, cytidine, phosphoric esters of cytidine, deoxy cytidine, phosphoric esters of deoxy cytidine, and salts thereof; uracil, uridine, phosphoric esters of uridine, metabolic products of phosphoric esters of uridine, and salts thereof; and thymine, thymidine, phosphoric esters of thymidine, orotic acid, 5'-orothidic acid, and salts thereof.

Also, the salts may include alkali metal salts, such as sodium and potassium salts; basic alkaline earth metal salts, such as calcium, magnesium and barium salts; basic amino acid salts; ammonium salts; alkanol amine salts, etc.

Also, the nucleic acids may be low-molecular-weight nucleotides having a molecular weight of 100,000-50,000,000 Da.

The concentration of nucleic acid in the composition may be 0.1-50%, and nucleic acid may be contained in an amount of 10-90 parts by weight based on 100 parts by weight of the pharmaceutical composition.

Nucleic acids are known to enhance humoral immunity, produce interleukin-2 (IL-2) and influence helper T-cell activity. Also, among nucleic acids, ATP and UTP are known to stimulate the proteoglycan and collagen aggregation and synthesize joint chondrocytes.

In addition, uridine and uridine derivatives are known to promote collagen biosynthesis in chondrocyte cells, fibroblast cells, synovial cells and the like.

The uridine derivatives include, but are not limited to, uracil, uridine monophosphate, uridine diphosphate, triacetyl uridine, tribenzoyl uridine, 5-ethyl uridine, 2-deoxyuridine, isopropylidene uridine, etc.

Also, the pharmaceutical composition of the present invention may further comprise vitamin C or its derivative which activates hydrolylase required for collagen synthesis. As described above, as the amount of vitamin C increases, the amount of hydroxyproline increases, and thus collagen synthesis is also promoted.

Vitamin C (or ascorbic acid) is known to delay the progression of osteoarthritis.

Vitamin C ascorbic acid) derivatives may be used alone or in combination and may include all enantiomers. Preferably, ascorbic acid may be provided in the L form (levo form). Examples of the vitamin C derivatives may include, but are not limited to, an alkyl ester of L-ascorbic acid; a phosphate of L-ascorbic acid; a sulfate of L-ascorbic acid; a sodium salt of L-ascorbic acid, etc.

Herein, the concentration of vitamin C in the composition may be 0.01-10%, and vitamin C may be contained in an amount of 0.1-50 parts bt weight based on the total weight of the pharmaceutical composition.

The pharmaceutical composition according to the present invention is used in the form of a medicament or a food. When it is used as a medicament, it may be administered by various general routes, including oral administration, administration as fluid, and application or spray to the skin.

For oral administration, the pharmaceutical composition of the present invention may be used alone or as formulations, such as tablets, pills, capsules, gels or syrups, together with pharmaceutically acceptable carriers or excipients. Specifically, the composition may be administered as aqueous solutions together with additives such as salts, buffers or chelating agents. The carriers include, but are not limited to, saline, buffered saline, water, ethanol, etc., and all suitable preparations known in the art may be used in the present invention (Remington's Pharmaceutical Science, Mack Publishing Company, Easton PA).

Also, drage cores may be provided with suitable coatings using concentrated sugar solutions which may contain Arabic gum, talc, polyvinylpyrrolidone, carbopol gel, polyethylene glycol and/or titanium dioxide, or coating solutions in suitable organic solvents or solvent mixtures.

Pharmaceutical compositions which can be used for oral administration include push-fit capsules made of gelatin and soft capsules made of gelatin and a plasticizer such as glycerol. The push-fit capsules may comprise, in addition to active ingredients, fillers such as lactose, binders such as starch, lubricants such as magnesium stearate, or stabilizers. The soft capsules can be prepared by dissolving or suspending active compounds in suitable liquids, such as fatty oil, liquid paraffin or liquid polyethylene glycol.

Also, the composition may be used as targeted drug delivery systems, such as liposome coated with a specific antibody targeting arthritis or fibrosis tissue.

For administration by injection, the composition may be added to a suitable buffer or isotonic agent and dissolved in sterile distilled water, and it may be injected directly into the spinal cavity, connective tissue or vein. When the pharmaceutical composition is administered by injection, it will change the osmotic environment in cells to influence gene expression. For example, in intervertebral disc cells, the composition administered by injection will increase the expression of aggrecan and type II collagen as the osmotic pressure increases.

When the composition of the present invention is used as foods, it may be used in the form of medical foods or health foods, in addition to conventional foods or beverages, which will show the above-described effects.

### Mode for Invention

Hereinafter, the present invention will be described in further detail with reference to examples. However, the scope of the present invention is not limited to these examples.

### Example 1: Preparation of therapeutic compositions

The therapeutic compositions shown in Table 1 below were prepared. The components shown in Table 1 were all purchased from Sigma Chemical Co.(USA).

**[Table 1]**

| Component (concentration %) | | Preparation 1 | Preparation 2 | Preparation 3 | Preparation 4 |
|---|---|---|---|---|---|
| Sorbitol (10%) | | 1 | 5 | 10 | 20 |
| Ornithine (50%) | | 5 | 15 | 22.5 | 40 |
| Amino acid (20%) | Glycine | 0.5 | 4 | 10.25 | 20 |
| | Proline | 0.5 | 3 | 8.75 | 15 |
| | Hydroxyproline | 0.5 | 3 | 8.75 | 15 |
| | Arginine | 0.5 | 3 | 8.75 | 15 |
| | Glutamic acid | 0.1 | 2 | 2.55 | 5 |
| | Glutamine | 0.1 | 2 | 2.55 | 5 |
| | Aspartic acid | 0.1 | 2 | 2.55 | 5 |
| | Cysteine | 0.1 | 1 | 1.55 | 3 |
| | Methionine | 0.1 | 1 | 1.55 | 3 |
| | Serine | 0.1 | 1 | 1.55 | 3 |
| Vitamin C (5%) | | 0.1 | 1 | 2.55 | 5 |
| LMW DNA(1%) | 0.1 | 10 | 25 | 50 | |

### Example 2: Construction of test animal model

In this experiment, a rabbit model having a structure similar to the human knee joint was used. Specifically, 11 New Zealand white rabbit (average weight: 3.0±0.01kg (2.8~2.9kg), 8-month old) were used. The reason why the mature rabbits were used is that osteoarthritis in the rabbits is induced in conditions similar to those for humans and that the ability to regenerate cartilage is inferior to that of immature rabbits.

The 11 test rabbits were systemically anesthetized and the knee joint of the right hind leg of the rabbits was subjected to cruciate ligament resection, after which the rabbits were allowed to stand for 6 weeks, thereby inducing arthritis in the rabbits.

Among these test rabbits, 6 rabbits were used as a test group. 6 weeks after the cruciate ligament resection, the test rabbits were systemically anesthetized, and then the therapeutic composition of preparation 2 prepared in Example 1 was injected into the articular cavity of the arthritis-induced right knee joint of the test group rabbits. The therapeutic composition of preparation 2 of Example 1 was injected a total of 5 times at 2-week intervals.

Also, among the test rabbits, 5 rabbits were used as a control group. In order to give the same stress as the test group, the same amount of physiological saline as the test composition was injected a total of 5 times at 2-week intervals from 2 weeks after the cruciate ligament resection.

In addition, the left knee joint which was not subjected to cruciate ligament resection and other surgical operations was used as a normal group.

### Example 3: Measurement of regeneration and repair of cartilage tissue

4 weeks after the final injection of the therapeutic composition of preparation 2 of Example 1, the test rabbits were systematically anesthetized and euthanized, and both the left and right knee joints were collected, immersed and fixed in 10% neutral formalin (pH 7.4) for 24 hours or more.

The collected left and right knee joints of the test group and the control group evaluated by histopathological evaluation methods (H&E staining, modified Mankin's grading method), and the degrees of breakage and regeneration of the joint cartilages were compared between the test group and the control group.

Also, in order to evaluate the state of the normal left keee joint cartilage (normal group), the state of the right knee cartilage of the test group and the control group was corrected, and then the difference between the groups was statistically evaluated.

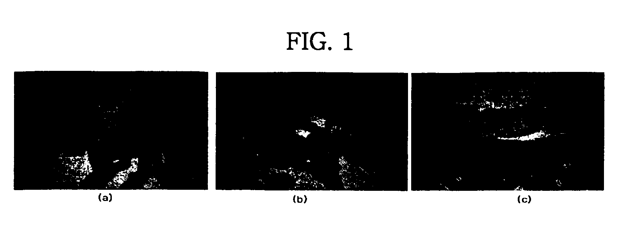

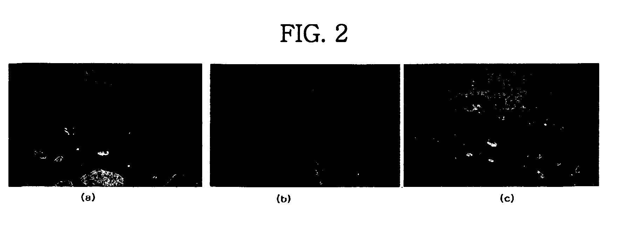

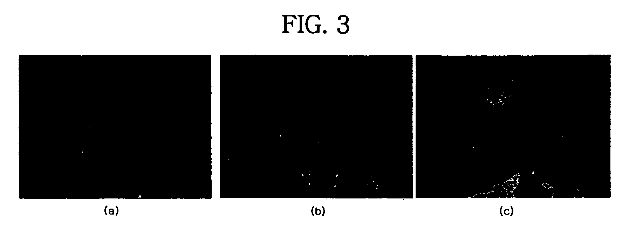

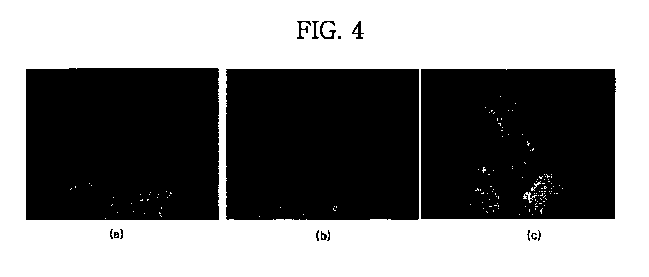

FIGS. 1 to 4 show the results of observing the effects of the therapeutic composition of the present invention in tissue. Specifically, the knee joint cartilage tissue was resected from the test rabbits and embedded in paraffin, followed by serial sectioning. Then, the cartilage tissue was stained with hematoxylin-eosin (H&E) and observed with an optical microscope at 100x magnification. As a result, as can be seen in FIGS. 1 to 4, the test group did not generally differ from the normal group, even though a portion of the surface structure of the joint cartilage in the test group was irregular. Also, the state of cells in the test group was good. However, in the control group in comparison with the normal group and the test group, the surface of the cartilage tissue was significantly irregular, and pannus formation and clefts to the transitional or radial zone were observed. In addition, in the control group, cell hypercellularity and cloning were observed, suggesting that the injury of joint cartilage in the control group was significantly serious compared to those in the normal group and the test group. Table 3 below shows the results of evaluating the femoral condyle surface, tibial plateau surface and total cartilage score of the test group and control group of Example 2 at 18 weeks after start of the experiment using the modified Mankin's grading method.

**[Table 2]**

| | | | | | |
|---|---|---|---|---|---|
| Criteria for histological evaluation of joint cartilage tissue by modified Mankin's grading method (Gerwin N, Hops C, Lucke A. Intraarticular grug delivery in osteoarthritis. Adv Drug Rev 2006; 58:226-242) | | | | | |

| Item | Grade | Evaluation | Item | Grade | Evaluation |
|---|---|---|---|---|---|
| Tissue | 0 | Normal | Cell state | 0 | Normal |
| | | | | 1 | Diffuse hypercellularity |
| | 1 | Irregular surface | | 2 | Cloning) |
| | 2 | Pannus formation and surface irregularities | | 3 | Hypocellularity) |
| | 3 | Clefts to transitional zone | | | |
| | 4 | Clefts to radial zone | | | |
| | 5 | clefs to calcified zone | | | |
| | 6 | Complete disorganization | | | |

**[Table 3]**

| Evaluated site | Normal group | Test group | Control group | P |
|---|---|---|---|---|
| Femoral condyle surface | 1.14±0.52 a | 3.67±0.76 b | 5.80±0.49 c | 0.000 |
| Tibial plateau surface | 1.18±0.35 a | 2.17±0.58 a | 3.60±0.50 b | 0.000 |
| Total cartilage score | 1.16±0.31 a | 2.92±0.49 b | 4.70±0.42 c | 0.000 |

In Table 3 above, the evaluated grade scores were expressed as mean ± SD and compared between the groups by ANOVA and Duncan tests using SPSS 17.0 version. P values p<0.05 were considered statistically significant. In Table 3, higher values mean more severe breakage of joint cartilage, groups indicated by the same alphabet indicate that the difference between these groups is not significant, and groups indicated by different alphabets indicate that the difference between these groups is significant. In the results of statistical analysis, the score of evaluation for femoral condyle surface was higher in order of the normal group > the test group > the control group (p=0.000). The score of evaluation for tibial plateau surface was substantially similar between the normal group and the test group, but was significantly the highest in the control group (p=0.000). In addition, the total cartilage score was higher in order of the normal group > the test group > the control group (p=0.000). All the above results were statistically significant (P<0.05). Thus, it was found that the therapeutic composition of the present invention had the effect of inhibiting the progression of arthritis in the femoral and tibidal sites and that the effect of the composition was particularly significant in the tibia on which greater weight bearing is placed, suggesting that the cartilage in the test group was repaired to the same level as that of the normal group.

## Claims

1. A pharmaceutical composition for preventing and treating joint-related diseases and connective tissue-related diseases, the composition comprising two or more selected from the group consisting of polyol, amino acid, ornithine, nucleic acid and vitamin C.

2. The pharmaceutical composition of claim 1, wherein a concentration of the polyol in the composition is 2.5-50%, and the polyol is contained in an amount of 10-90 parts by weight based on 100 parts by weight of the pharmaceutical composition.

3. The pharmaceutical composition of claim 1, wherein the amino acid comprises one or more selected from the group consisting of proline, glycine, glutamine, alanine, aspartic acid, serine, glutamic acid, methionine, lysine, cysteine, hydroxyproline and arginine, a concentration of the amino acid in the composition is 2.5-50%, and the amino acid is contained in an amount of 10-90 parts by weight based on 100 parts by weight of the pharmaceutical composition.

4. The pharmaceutical composition of claim 3, wherein the amino acid comprises, based on a total weight of the amino acid, 5-95 parts by weight of proline, 5-95 parts by weight of hydroxyproline, 5-95 parts by weight of glycine, 1-50 parts by weight of glutamine, 1-30 parts by weight of alanine, 1-30 parts by weight of aspartic acid, 1-30 parts by weight of serine, 5-95 parts by weight of arginine, 1-50 parts by weight of methionine, 1-30 parts by weight of cysteine, 1-30 parts by weight of lysine, and 1-30 parts by weight of glutamic acid.

5. The pharmaceutical composition of claim 3, wherein the amino acid comprises, based on a total weight of the amino acid, 5-85 parts by weight of proline, 5-85 parts by weight of hydroxyproline, 10-90 parts by weight of glycine, 1-30 parts by weight of glutamine, 1-20 parts by weight of alanine, 1-20 parts by weight of aspartic acid, 1-20 parts by weight of serine, 5-80 parts by weight of arginine, 1-30 parts by weight of methionine, 1-20 parts by weight of cysteine, 1-20 parts by weight of lysine, and 1-20 parts by weight of glutamic acid.

6. The pharmaceutical composition of claim 3, wherein the amino acid comprises, based on a total weight of the amino acid, 5-70 parts by weight of proline, 5-70 parts by weight of hydroxyproline, 10-70 parts by weight of glycine, 1-20 parts by weight of glutamine, 1-10 parts by weight of alanine, 1-10 parts by weight of aspartic acid, 1-10 parts by weight of serine, 5-60 parts by weight of arginine, 1-15 parts by weight of methionine, 1-10 parts by weight of cysteine, 1-10 parts by weight of lysine, and 1-10 parts by weight of glutamic acid.

7. The pharmaceutical composition of claim 1, wherein a concentration of the ornithine in the composition is 2.5-65%, and the ornithine is contained in an amount of 10-90 parts by weight based on 100 parts by weight of the pharmaceutical composition.

8. The pharmaceutical composition of claim 1, wherein the nucleic acid comprises at least one of a purine-based nucleic acid-associated substance and a pyrimidine-based nucleic acid-associated substance, a concentration of the nucleic acid in the composition is 0.1-50%, and the nucleic acid is contained in an amount of 10-90 parts by weight based on 100 parts by weight of the pharmaceutical composition.

9. The pharmaceutical composition of claim 8, wherein a molecular weight of the nucleic acid is 100,000 Da-50,000,000 Da.

10. The pharmaceutical composition of claim 8 or 9, wherein the purine-based nucleic acid-associated substance is one or more selected from the group consisting of adenine, adenosine, phosphoric esters of adenosine, metabolic products of phosphoric esters of adenosine, and salts thereof, and guanine, guanosine, phosphoric esters of quanosine, metabolic products of phosphoric esters of guanosine, and salts thereof.

11. The pharmaceutical composition of claim 8 or 9, wherein the pyrimidine-based nucleic acid-associated substance comprises one or more selected from the group consisting of cytosine, cytidine, phosphoric esters of cytidine, deoxy cytidine, phosphoric esters of deoxy cytidine, and salts thereof; uracil, uridine, phosphoric esters of uridine, metabolic products of phosphoric esters of uridine, and salts thereof; and thymine, thymidine, phosphoric esters of thymidine, orotic acid, 5'-orothidic acid, and salts thereof.

12. The pharmaceutical composition of claim 1, wherein the vitamin C includes a vitamin C derivative, the concentration of the vitamin C in the composition is 0.01-10%, and the vitamin C is contained in an amount of 0.1-50 parts by weight based on 100 parts by weight of the pharmaceutical composition.

13. The pharmaceutical composition of claim 12, wherein the vitamin C derivative comprises one or more selected from the group consisting of an alkyl ester of L-ascorbic acid, a phosphate of L-ascorbic acid, a sulfate of L-ascorbic acid, and a sodium salt of L-ascorbic acid.

14. A pharmaceutical composition for preventing and treating joint-related diseases and connective tissue-related diseases, the composition comprising: 20-80 parts by weight of polyol whose concentration in the composition is 2.5-50%; 20-80 parts by weigh of ornithine whose concentration in the composition is 2.5-50%; 20-80 parts by weight of amino acid whose concentration in the composition is 2.5-50%, the amino acid being one or more selected from the group consisting of proline, glycine, glutamine, alanine, aspartic acid, serine, glutamic acid, methionine, lysine, cysteine, hydroxyproline and arginine; 20-80 parts by weight of nucleic acid whose concentration in the composition is 0.1-50%; and 0.1-15 parts by weight of vitamin C whose concentration in the composition is 0.01-10%.

15. The pharmaceutical composition of claim 14, wherein a molecular weight of the nucleic acid is 100,000 Da-50,000,000 Da.
